# EUROPEAN PATENT APPLICATION

(11) **EP 3 838 141 A1**
(43) Date of publication of application: **23.06.2021**
(21) Application number: 19217083.5
(22) Date of filing: 17.12.2019
(51) Int. Cl.: A61B 5/11, A61B 5/00

(54) **SYSTEM AND METHOD TO DETERMINE IF A DEVICE IS WORN ON A SUBJECT'S DOMINANT LIMB**

(71) Applicant: Koninklijke Philips N.V., 5656 AG Eindhoven (NL)
(72) Inventor: SAPORITO, Salvatore, 5656 AE Eindhoven (NL); RISPENS, Sietse, 5656 AE Eindhoven (NL)
(74) Representative: Philips Intellectual Property & Standards

(57) **Abstract**

Proposed are concepts for determining if a device is being (or has been) worn on the dominant or non-dominant limb of a subject. Such concepts may be based on comparing sensed movements of the device being worn by the user with sensed movements of another, supplementary device worn or carried by the subject. By way of example, a proposed embodiment may enable determination of whether a wrist-wearable device is being worn on the dominant hand of a subject by comparing the movements of the wrist-wearable device (e.g. as detected by an accelerometer of the device) with movement of a portable computing device also carried or worn by the subject.

## Description

### FIELD OF THE INVENTION

The invention relates to the field of wearable devices, and more particularly to determining if a device is worn on a subject's dominant limb

### BACKGROUND OF THE INVENTION

Typically, a subject will have a preference for use of a limb, known as the dominant limb, with the less preferred limb being then the non-dominant limb. For example, a subject may prefer to use his/her right hand for performing certain actions or activities, thus resulting in that user's right hand being the user's dominant hand and the user's left hand being the user's non-dominant hand.

Models based on detected physiological signals (e.g. energy expenditure, fall detection, optical heart rate detection, blood pressure estimation, step counting...) have been developed assuming device being worn on the dominant (or non-dominant) arm. However, research has shown that there is no significant difference in the total activity level between the dominant and non-dominant limb.

In clinical applications, a subject's dominant limb is typically determined by questionnaires (that may not be accurate). Strength tests are sometimes used, but limited in availability and practicality.

One known approach to detecting whether a device is being worn on a subject's dominant or non-dominant limb is to employ sensor data analysis combined with available information about the limb preference of the user. However, this has the drawback that such information about limb preference is typically unavailable. The subject may therefore have to manually provide such information, which may be inconvenient and/or inaccurate.

### SUMMARY OF THE INVENTION

The invention is defined by the claims.

According to examples in accordance with an aspect of the invention, there is provided a computer-based method for determining if a wearable device is worn on a subject's dominant limb, the wearable device comprising a motion sensor configured to generate movement data representative of movement of the wearable device.

The method comprises: identifying a time period of interest during which the subject is both: wearing the wearable device; and carrying a supplementary device comprising a supplementary motion sensor configured to generate supplementary movement data representative of movement of the supplementary device; obtaining movement data representative of movement of the wearable device during the identified time period; obtaining supplementary movement data representative of movement of the supplementary device during the identified time period; determining a first characteristic of movement based on the obtained movement data; determining a second characteristic of movement based on the obtained supplementary movement data; and based on a comparison of the first and second characteristics of movement, determining if the wearable device is worn on the subject's dominant limb during the identified time period.

Proposed are concepts for determining if a device is being (or has been) worn on the dominant or non-dominant limb of a subject. Such concepts may be based on comparing sensed movements of the device being worn by the user with sensed movements of another, supplementary device worn or carried by the subject. By way of example, a proposed embodiment may enable determination of whether a wrist-wearable device is being worn on the dominant hand of a subject by comparing the movements of the wrist-wearable device (e.g. as detected by an accelerometer of the device) with movement of a portable computing device (e.g. smartphone or tablet computer) also carried or worn by the subject (e.g. as detected by an accelerometer of the portable computing device).

Embodiments may therefore leverage movement data obtained from two different motion sensors (e.g. two accelerometers) worn or carried by the same subject.

Such embodiments are based on the realisation that a subject may typically carry a portable computing device that includes one or more motion sensors. Widely available and carried devices may thus provide supplementary movement data that can be used in combination with movement data supplied by the wearable device. For instance, it may be determined if the wearable device is worn on the subject's dominant limb (e.g. hand or foot) by assessing correlations between the movement data (from the wearable device) and the supplementary movement data (from the supplementary device carried by the same subject).

It has been realized that the nature of such correlations made depend on type of wearable device and type of supplementary device.

Specifically, where the supplementary device is carried or worn on a subject's torso, it is proposed that poor correlation (i.e. low similarity) between a characteristic of the concurrent (i.e. simultaneous) movement of the wearable device and the supplementary device may be indicative of the wearable device being worn on the dominant limb of the subject. This is based on the proposal that a dominant limb will be preferred for instrumental, goal-oriented Activities of Daily Living (ADLs) and thus move differently from the body of the subject during performance of an ADL. For instance, from identifying dissimilar simultaneous movement of the wearable device and the supplementary device, it may be inferred that the wearable device is worn on dominant limb of the subject.

Conversely, where the supplementary device is used to perform an action, it is proposed that strong correlation (i.e. high similarity) between a characteristic of the concurrent (i.e. simultaneous) movement of the wearable device and the supplementary device may be indicative of the wearable device being worn on the dominant limb of the subject. This is based on the proposal that a dominant limb will be preferred for using the supplementary device to perform an action and thus move in the same manner as the dominant limb during performance of the action. For instance, from identifying similar simultaneous movement of the wearable device and the supplementary device, it may be inferred that the wearable device is worn on dominant limb of the subject. For example, such a proposal may exploit the fact that, when a smartphone is carried by the subject, the dominant arm is statistically likely to be the arm that is used to perform an action with the smartphone.

Embodiments may therefore provide a simple and accurate approach to determining if a wearable device is worn on a subject's dominant limb. Further, the approach may make use of data and functionalities available from existing portable computing devices that are widely available and routinely carried by subjects.

Purely by way of example, a proposed embodiment may determine if a wristband device is worn on a subject's dominant wrist hand based on sensed movements of the wristband device and sensed (concurrently occurring) movements of a mobile phone carried by the subject.

Furthermore, proposed embodiment may be configured to repeatedly (e.g. periodically, such as every hour or every day) determine if a wearable device is worn on a subject's dominant limb. Multiple determination may therefore be obtained, and these multiple determinations may be combined into a final (i.e. combined or collective) determination for a specific period of interest (e.g. a day combining hourly information). This may implemented, for instance, by considering majority voting results over a subset of dominant/non-dominant estimates.

In some embodiments, determining if the wearable device is worn on the subject's dominant limb during the identified time period may comprise: comparing the first and second characteristics of movement to determine a measure of similarity between the first and second characteristics of movement; and determining if the wearable device is worn on the subject's dominant limb during the identified time period based on the determined measure of similarity. A simple comparison process may therefore be employed to infer whether nor the wearable device is worn on the subject's dominant limb. In this way, the cost and/or complexity of proposed embodiments can be reduced.

For instance, determining if the wearable device is worn on the subject's dominant limb during the identified time period may comprise: determining the wearable device is worn on the subject's dominant limb during the identified time period if the determined measure of similarity is below a predetermined threshold value. In other words, responsive to determining that the measure of similarity is outside a predetermined range (e.g. a range within which the first and second characteristics of movement are considered to be similar, i.e. closely correlated), it may be inferred that the wearable device is on the subject's dominant limb. Conversely, if the measure of similarity has a high value (e.g. is equal to or exceeds the predetermined threshold), it may be inferred that the wearable device is not on the subject's dominant limb. Proposed embodiments may therefore employ a relatively simple classification process based on a measure of similarity of simultaneous movement of the wearable device and the supplementary device (e.g. sensed movement of the wearable device and the supplementary device when both being carried/worn by the subject).

By way of example, determining a measure of similarity may comprise determining at least one of: correlation coefficients; rank correlation coefficients; a sum of absolute differences; norm of vectors; differences in timestamps of movement events; orientation of a three-dimensional vectors; and differences in orientation of three-dimensional vectors. Various properties or characteristics of sensed movement may therefore be used to describe and compare the sensed movement of the wearable device and the supplementary device. Proposed embodiments may therefore leverage a realisation that similarity of sensed movement of the wearable device and a supplementary device carried/worn by the user may be indicative of whether or not the wearable device is on the subject's dominant limb. By using various algorithms and/or techniques for quantifying and comparing similarity between sensed movement data, embodiments may facilitate simple but accurate determinations to be made.

In some embodiments, identifying a time period of interest may comprise identifying the time period of interest responsive to the occurrence of a predetermined action performed by the subject. For example, where the supplementary device comprises a portable computing device, the predetermined action may comprise a gesture using an input interface of the portable computing device. Such an exemplary gesture may comprise a single or multi-part movement of the hand of a subject which performs an action such as: device-unlock/lock; application execution; button activation/press; side/rocker button press; device pick up; input typing; peripheral connection/disconnection; touchscreen use; screen rotation from portrait to landscape mode; call mode activation; fingerprint sensor use; or light/camera sensor uses for photo-plethysmography applications.

A characteristic of movement may comprise: amplitude of movement; direction of movement; frequency of movement; speed of movement; variation in position; variation in angle; high-level descriptive characteristics such as classified activities (e.g., walking, cycling, standing, sitting, lying); variance rate (e.g. position/speed variation per second); movement onset and/or conclusion times; times of specific local maxima or minima; difference in the angle between the wearable device and supplementary device orientations (e.g. certain axis of smartwatch and smartphone may be aligned during call only). Examples may therefore relate to, and thus be derived from a combination of information from, both devices. For such example, when a characteristic of movement is derived from a single axis of an accelerometer arrangement, a preliminary preprocessing step may be employed to determine the correspondence between the axes of the two devices.

Various properties or characteristics of the sensed movement may therefore be used by embodiments. This may enable multiple comparisons to be made, for example, so as to facilitate the checking, qualification and/or refinement of established results.

It is also noted that embodiments may include the concept of modifying an operation or behavior of the wearable device, based on the result of determining if the wearable device is worn on the subject's dominant limb during the identified time period. For instance, the display of information by the wearable device may be made dependent on whether or not it is worn on the dominant limb of a user. By way of further example, an application or notification may be automatically provided the subject in response to the wearable device being determined as being worn on a subject's dominant wrist/hand. Also, in some embodiments, a function or algorithm performed by the wearable device may be adapted based on the result of determining if the wearable device is worn on the subject's dominant limb. For instance, a parameter of a detection or monitoring function provided by the wearable device may be adapted to account for the wearable device not being worn on a subject's dominant wrist.

In some embodiments, the motion sensor may comprise an accelerometer arrangement. Similarly, the supplementary motion sensor may comprise an accelerometer arrangement (i.e. a second accelerometer arrangement). Simple, cheap, and/or widely available motion sensor technologies may therefore be employed by embodiments, thus making proposed embodiments cheap to implement. Indeed, embodiments may make use of motion sensors already present in wearable devices and portable computing devices (such a smartphone or tablet computer).

It will be appreciated that proposed embodiments may be configured to simply obtain (e.g. receive or retrieve) movement data and supplementary data that the wearable and supplementary devices are already configured to generate. Such embodiments may therefore be used in conjunction with devices that are already available, and thus provide supplementary functionality. For example, proposed embodiments may be used in combination with a conventional smart watch and smart phone that are both already used (e.g. worn and/or carried) by a subject. In this way, embodiments may increase a value of existing/conventional wearable device and/or systems employing such a device by providing modified and/or extended functionality.

Conversely, other embodiments may include at least one of the wearable device and the supplementary device. Thus, rather than relying on obtaining data from separate devices, embodiments may be configured to include at least one of the device and/or motion sensors required in order to provide movement data and/or supplementary movement data. For instance, an embodiment may include the wearable device having a motion sensor (such as an accelerometer arrangement). Embodiments may thus provide a subject monitoring system or a PERS system with improved or extended functionality provided as a result of the proposed concept(s). Purely by way of further example, an embodiment may comprise a PERS system that is adapted to determine if a wearable device is worn on a monitored subject's dominant limb.

According to another aspect of the invention, there is provided a computer-based method for determining a subject's limb preference for wearing a wearable device. The method comprises: determining if a wearable device is worn on the subject's dominant limb according to a proposed embodiment, obtaining an indication of a current wearing position of the wearable device on the subject; and determining the subject's limb preference for wearing a wearable device based on: the result of determining if the wearable device is worn on the subject's dominant limb; and the obtained indication of a current wearing position of the wearable device on the subject.

Thus, it is proposed to leverage results obtained by a proposed embodiment to provide a more reliable and/or accurate determination of a subject's limb preference. In this way, there may be provided an automatic way to obtain an accurate indication of a subject's limb preference without relying on potentially inaccurate or misleading information provided by the subject (e.g. in response to a questionnaire).

According to examples in accordance with an aspect of the invention, there is provided a computer program comprising code means for implementing the method of any previously described method when said program is run on a processing system.

According to a concept of the invention, there is provided a system for determining if a wearable device is worn on a subject's dominant limb, the wearable device comprising a motion sensor configured to generate movement data representative movement of the wearable device. The system comprises: an analysis unit configured to identify a time period of interest during which the subject is both: wearing the wearable device; and carrying a supplementary device comprising a supplementary motion sensor configured to generate supplementary movement data representative of movement of the supplementary device; an interface component configured to obtain movement data representative of movement of the wearable device during the identified time period, and to obtain supplementary movement data representative of movement of the supplementary device during the identified time period; a processing unit configured to determine a first characteristic of movement based on the obtained movement data, and to determine a second characteristic of movement based on the obtained supplementary movement data; and a determination unit configured to determine, based on a comparison of the first and second characteristics of movement, if the wearable device is worn on the subject's dominant limb during the identified time period.

There is also proposed a system for determining a subject's limb preference for wearing a wearable device. The system comprises a system for determining if a wearable device is worn on the subject's dominant limb according to a proposed embodiment; an input interface configured to obtain an indication of a current wearing position of the wearable device on the subject; and a decision unit configured to determine the subject's limb preference for wearing a wearable device based on: the result of determining if the wearable device is worn on the subject's dominant limb; and the obtained indication of a current wearing position of the wearable device on the subject.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIGS. 1A-1B illustrate examples of simultaneously acquired accelerometer signals from a wrist-worn device and an accelerometer (included in a smartphone) being picked up from a subject's pocket, used for a short time and then put back in the pocket;
FIG. 2A illustrates an example of simultaneously acquired (x, y, and z-axis) accelerometer signals from a wrist-worn device and an accelerometer (included in a smartphone) being picked up from the subject's pocket, used for a short time and then put back in the pocket;
FIG. 2B illustrates an example of simultaneously acquired accelerometer signals from a wrist-worn device (worn on the subject's dominant arm) and an accelerometer (included in a smartphone), wherein the subject was repeatedly unlocking and locking the smartphone;
FIG. 2C illustrates an example of simultaneously acquired accelerometer signals from a wrist-worn device (worn on the subject's dominant arm) and an accelerometer (included in a smartphone), wherein the subj ect typing text using the smartphone;
FIG. 3 depicted a set of graphs demonstrating a proposed concept for determining if a wearable device is worn on a subject's dominant limb;
FIG. 4 is a flow diagram of a method for determining if a wearable device is worn on a subject's dominant limb according to an embodiment;
FIG. 5 **Erreur ! Source du renvoi introuvable.** is a simplified block diagram of a system for determining if a wearable device is worn on a subject's dominant limb according to an embodiment; and
FIG. 6 illustrates a system for determining a subject's limb preference for wearing a wearable device according to a proposed embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

According to a concept of the invention, there is proposed a method and system for determining if a wearable device is worn on the dominant or non-dominant limb of a subject. The concept includes obtaining, for a time period of interest during which the subject is both wearing the wearable device and carrying a supplementary device: (i) movement data representative of movement of the wearable device and (ii) supplementary movement data representative of movement of the supplementary device. Using the obtained data, one or more characteristics of the movement of the wearable device and supplementary device are compared and, based on a result of the comparison(s), it is determined if the wearable device is worn on the subject's dominant limb during the identified time period.

Embodiments are at least partly based on the realization that movement of a supplementary device carried by the subject (such as a smartphone) for example may be sensed and then analyzed with respect to sensed movement of the wearable device. Such analysis may assess the similarity (or lack thereof) of movement of the wearable device and supplementary device and, based on the similarity, it may be deduced whether or not the wearable device is worn on the subject's dominant limb. In particular, it is proposed that low similarity between simultaneous movement of the wearable device and the supplementary device worn or carried on the body of the subject may indicate that the wearable device is worn on the dominant limb of the subject. This is based on a proposal that a dominant limb will be preferred for performing goal-oriented actions and/or goal-oriented Activities of Daily Living (ADLs), and thus the dominant limb will move differently from the body of the subject during performance of such an activity/ADL. For instance, responsive to identifying dissimilar simultaneous movement of the wearable device and the supplementary device, it may be determined that the wearable device is worn on a dominant wrist of the subject.

Illustrative embodiments may, for example, be employed in (or in conjunction with) conventional smartphones or tablet computers that already include one or more motion sensors and are typically carried by subjects.

Proposed is an approach for enabling the dynamic determination of whether a device is being (or has been) worn on the dominant or non-dominant limb of a subject. Such an approach may leverage movement data obtained from a supplementary device (having a motion sensor) that is carried by the subject, and then use this supplementary data with movement data obtained from a motion sensor of the wearable device. Such proposals may thus exploit that the subject may typically carry a supplementary device (e.g. portable computing device) that includes one or more motion sensors.

Embodiments of the present invention may therefore be directed toward improving the accuracy and/or functionality of a wearable devices. Such wearable devices may include wearable monitoring devices, including wearable devices used in subject monitoring systems. Thus, by way of example only, illustrative embodiments may be utilized in many different types of clinical, medical or subject-related environments, such as a hospital, doctor's office, ward, care home, person's home, etc.

As mentioned above, proposed concepts are at least partly based on the realization that correlation between at least one characteristic of concurrent (i.e. simultaneous) movement of a wearable device worn by a subject (e.g. monitored patient) and a supplementary device carried/supported somewhere else on the subject's body may be indicative of whether the wearable device is being worn on the dominant limb of the subj ect.

Purely by way of example, a subject may be wearing a wristband health-monitoring device whilst also carrying a smartphone in his/her pocket (i.e. a different position from the subject's wrist). In this way, the subject may effectively be simultaneously carrying two motion sensing arrangements during his/her normal daily life activities. The first motion sensing arrangement may be provided in the wristband health-monitoring device and thus generate movement data representative of movement of the wristband health-monitoring device, and this may be generally representative of movement of the subject's wrist or hand. The second motion sensing arrangement may be provided in the smartphone and thus generate movement data representative of movement of the smartphone, and this may be generally representative of movement of the subject's whole body.

An example embodiment then selects a time period of interest. By way of example, the time period of interest may be identified in response to detecting the occurrence of a certain event, such as a device screen unlock or a predetermined type or pattern of movement. Other approaches to identifying a time period of interest may be based on using other signals or information, such as an activation signal from either device or a user interaction with either device.

The embodiment then obtains the movement data and supplementary movement data generated for the time period of interest. Also, it may be checked to determine if the time period is one during which the subject was wearing/carrying both devices.

From the obtained movement data and supplementary movement data, a characteristic of the movement (e.g. amplitude of movement, direction of movement, frequency of movement, variation in position, etc.) during the time period is determined for both devices. Using the determined characteristics, the movement is compared to assess a level of similarity between movement of the two devices.

Where the supplementary device is carried or worn close to a subject's torso, it is proposed that poor correlation (i.e. low similarity) between a characteristic of the concurrent (i.e. simultaneous) movement of the wearable device and the supplementary device may be indicative of the wearable device being worn on the dominant limb of the subject. This is based on the proposal that a dominant limb will be preferred for instrumental, goal-oriented Activities of Daily Living (ADLs) and thus move differently from the body of the subject during performance of an ADL.

For such cases of using the supplementary device to provide information about movement of the subject's body/torso, if the similarity (e.g. correlation) between movement of both devices is outside a certain pre-determined range during the selected periods (e.g. below a threshold value indicating a boundary between high and low similarity), then the wristband health-monitoring device is assumed to be performing instrumental, goal-oriented movements, and the user is determined to be wearing the wristband health-monitoring device on the dominant arm.

By way of further example, one may now consider an embodiment based on identifying a preference in using one of the two arms by a subject. This preference will be effective mainly for instrumental, goal-oriented ADLs (e.g. eating, using scissors, drinking from a cup, etc.). However, when the subject's entire body moves (e.g. during a walk, travelling) then both the dominant and non-dominant arm will move, undergo similar (i.e. highly correlated) movement to that of the subject's body. Based on such a proposal, inferences regarding detected movement and use of a dominant or non-dominant limb for comparison between limb motion and body/torso movement may be as summarised in the following table (Table 1):

**Table 1**

| | Dominant arm | Non-dominant arm |
|---|---|---|
| Instrumental activity (supplementary device measures low activity) | Motion is different - low correlation | Motion is similar - high correlation |
| Whole-body motion (supplementary device measures high activity) | Motion is similar - high correlation | Motion is similar - high correlation |
| Still (supplementary device measures low activity) | No motion - high correlation | No motion - high correlation |

Conversely, where the supplementary device is used to perform an action, it is proposed that strong correlation (i.e. high similarity) between a characteristic of the concurrent (i.e. simultaneous) movement of the wearable device and the supplementary device may be indicative of the wearable device being worn on the dominant limb of the subject. This is based on the proposal that a dominant limb will be preferred for using the supplementary device to perform an action and thus move in the same manner as the dominant limb during performance of the action.

For such cases of using the supplementary device to provide information about movement of a subject's limb, if the similarity (e.g. correlation) between movement of both devices is within a certain pre-determined range during the selected periods (e.g. above a threshold value indicating a boundary between high and low similarity), then the wristband health-monitoring device is assumed to be moving as a result of the subject performing an action on/with the supplementary device, and the user is determined to be wearing the wristband health-monitoring device on the dominant arm.

By way of further example, one may now consider an embodiment based on identifying a preference in using one of the two arms by a subject. This preference will be effective mainly for performing actions (e.g. scrolling, button pressing, swiping, executing an application/function, etc.) on or with the supplementary device, such as a smartphone. When the subject performs such an action with the supplementary device, the non-dominant and dominant arms will move differently and undergo dissimilar (i.e. poorly correlated) movement.

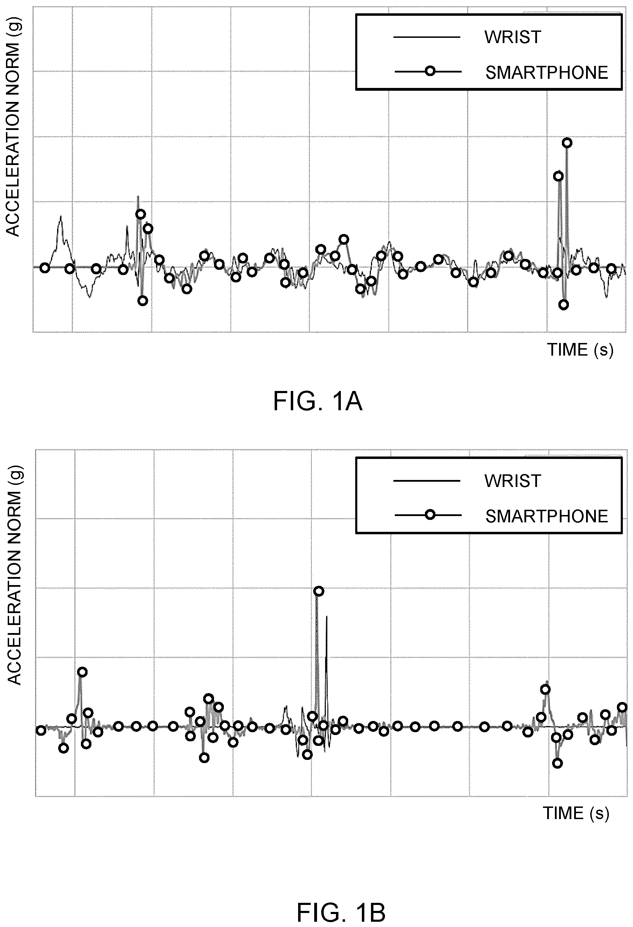

Such a proposal is illustrated by Figures 1A-1B which illustrate examples of simultaneously acquired accelerometer signals from a wrist-worn device and an accelerometer (included in a smartphone) being picked up from the subject's pocket, used for a short time and then put back in the pocket. The x-axis represents elapsed time in seconds (s) and the y-axis represents the detected acceleration norm (g). The line plot devoid of markers represents the variation in detected acceleration norm with respect to time as detected by the wrist-worn device. The line plot with circular markers represents the variation in detected acceleration norm with respect to time as detected by the pocket-placed accelerometer. Specifically, Figure 1A illustrates the acquired accelerometer signals when the wrist-worn device was worn on the subject's dominant arm, whereas Figure 1B illustrates the acquired accelerometer signals when the wrist-worn device was worn on the non-dominant arm. From Figures 1A and 1B it is seen that only signals collected at smartphone and the wrist-worn device worn on the dominant arm (i.e. the signals of Figure 1A) look similar as they represent the same motion.

Based on such a proposal, inferences regarding detected movement and use of a dominant or non-dominant limb may be as summarised in the following table (Table 2):

**Table 2**

| | Dominant arm | Non-dominant arm |
|---|---|---|
| Limb-based interaction between user and supplementary device, which involve some movement of the supplementary device (e.g. touchscreen operation) | Motion is simultaneous - high correlation | Motion is incoherent - No motion |
| No device interaction | No conclusion or use rule of table above | No conclusion or use rule of table above |

For correlations summarised in Table 2 above, it may be preferable to evaluate them over time scales comparable with user interaction (e.g. seconds, minutes), whereas for correlation summarised in Table 1 it may be preferable to evaluate the correlations over a longer, more general time scale (e.g. minutes, hours, weeks ...).

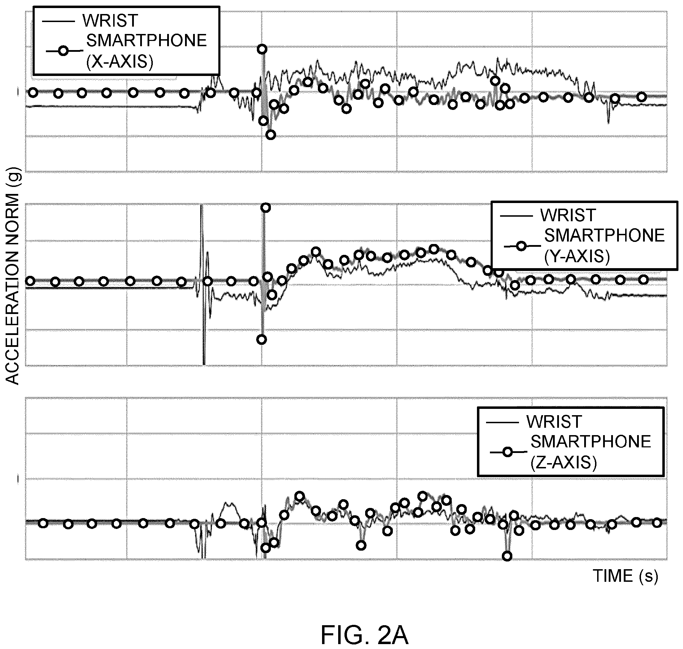

Further examples are illustrated by Figures 2A-2C. In each graph plot of Figures 2A-2C, the x-axis represents elapsed time in seconds (s) and the y-axis represents the detected acceleration norm (g). Also, the lines devoid of markers represent the variation in detected acceleration norm with respect to time as detected by the wrist-worn device. The line plots with circular markers represent the variation in detected acceleration norm with respect to time as detected by the pocket-placed accelerometer.

Figure 2A illustrates an example of simultaneously acquired (x, y, and z-axis) accelerometer signals from a wrist-worn device and an accelerometer (included in a smartphone) being picked up from the subject's pocket, used for a short time and then put back in the pocket. The top graph depicts the variation of the x-axis signals, the middle graph depicts the variation of the y-axis signals, and the bottom graph depicts the variation of the z-axis signals. Specifically, for the example of Figure 2A, the wrist-worn device was worn on the subject's dominant arm and the smartphone was picked up, and then rotated from portrait to landscape mode. The movement characteristic to compare for these signals may be the multiple accelerometer axis values and their low frequency component (which may represent smartphone orientation).

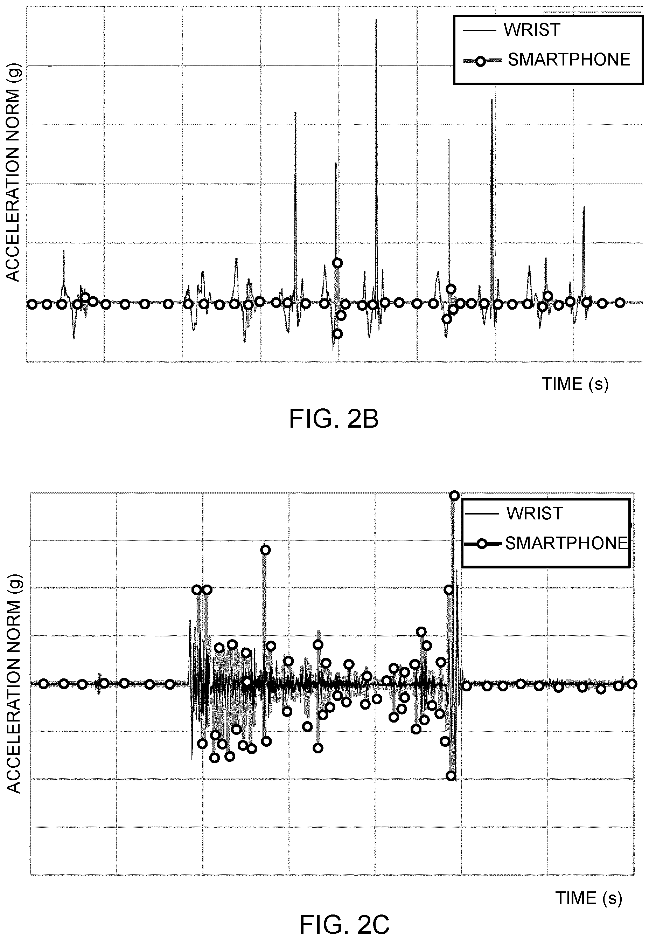

Figure 2B illustrates an example of simultaneously acquired accelerometer signals from a wrist-worn device (worn on the subject's dominant arm) and an accelerometer (included in a smartphone), wherein the subject was repeatedly unlocking and locking the smartphone. The movement characteristic to compare for these signals may be the timestamp values (i.e. timing) of peaks in the acceleration signal (i.e. local maxima).

Figure 2C illustrates an example of simultaneously acquired accelerometer signals from a wrist-worn device (worn on the subject's dominant arm) and an accelerometer (included in a smartphone), wherein the subject typing text using the smartphone.

From Figures 2A-2C it is seen that the signals collected at smartphone and the wrist-worn device worn on the dominant arm look similar as they represent the same motion.

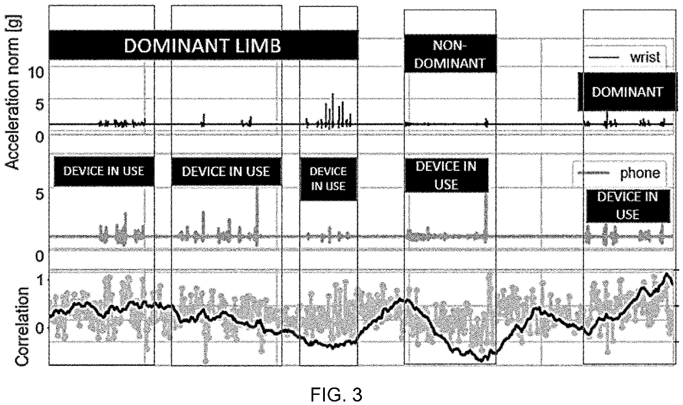

Referring now Figure 3, there is depicted a set of graphs demonstrating a proposed concept for determining if a wearable device is worn on a subject's dominant limb. The top graph depicts a variation of detected acceleration of wrist-worn device with respect to time, wherein the x-axis represents elapsed time in seconds (s) and the y-axis represents the detected acceleration norm (g). The middle graph depicts the simultaneous variation of detected acceleration of the subject's smartphone with respect to time, wherein the x-axis represents elapsed time in seconds (s) and the y-axis represents the detected acceleration norm (g). The bottom graph depicts, in light grey, the variation in the correlation coefficient between the norm of the accelerometer signals from the wrist-worn device and the smartphone, calculated in sliding windows (on groups of consecutive sample of each signal). The bottom graph also depicts, as a black line, the variation in the filtered correlation coefficient (e.g. mean correlation over the last 50 windows).

From the graphs of Figure 3, it is noticed that the correlation between the detected acceleration of the wrist-worn device and the smartphone drops when the wrist-worn device is worn on the non-dominant wrist and when device is in use (high movement level on phone).

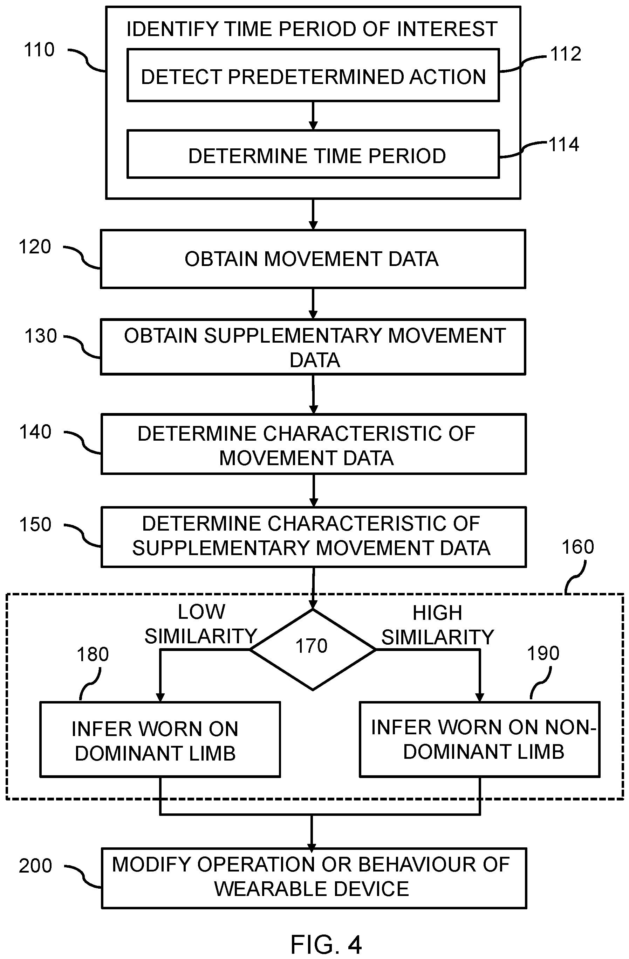

An example of a proposed embodiment will now be described with reference to Figure 4. Figure 4 is a flow diagram of a method for determining if a wearable device is worn on a subject's dominant limb according to an embodiment.

In this embodiment, the wearable device is a smartwatch running a health monitoring application and comprises a motion sensor. More specifically, the motion sensor comprises a (first) accelerometer arrangement configured to generate movement data representative of sensed movement of the smartwatch. Thus, the method of this embodiment is for determining if the smartwatch is worn on the subject's dominant wrist/hand.

Further, the subject carries a supplementary device, which is a portable computing device, specifically a smartphone (or mobile phone), comprising a (supplementary) motion sensor. More specifically, the motion sensor comprises a (second) accelerometer arrangement configured to generate supplementary movement data representative of sensed movement of the smartphone.

The method begins with step 110 of identifying a time period of interest during which the subject is both wearing/carrying the smartwatch and the smartphone. More specifically, in this exemplary embodiment, the step 110 comprises the sub-steps 112 and 114 of: detecting the occurrence of a predetermined action performed by the subject (step 112); and, responsive to detecting the occurrence of a predetermined action, determining the time period of interest. For instance, the predetermined action in this example comprises a gesture (e.g. swipe, touch movement, circular movement, multiple tap, etc.) using a touch sensitive input interface of the smartphone, and responsive to detecting the gesture, the time period is set to be the ten seconds immediately subsequent to the gesture (i.e. a time period of ten seconds starting at the time of gesture completion).

After identifying the time period, the method comprises step 120 of obtaining movement data representative of movement of the smartwatch during the identified time period. Here, the movement data is provided via a (wired or wireless) communication link established with the smartwatch. Next, step 130 comprises obtaining supplementary movement data representative of movement of the smartphone during the identified time period. Here, the movement data is provided via a (wired or wireless) communication link established with the smartphone.

The method then proceeds to step 140 of determining a (first) characteristic of movement based on the obtained movement data. Here, the characteristic of movement comprises a measure of at least one of: amplitude of movement; direction of movement; frequency of movement; speed of movement; variation in position; variation in angle; high-level descriptive characteristics such as classified activities (e.g., walking, cycling, standing, sitting, lying); variance rate (e.g. position/speed variation per second); movement onset and/or conclusion times; times of specific local maxima or minima; difference in the angle between the wearable device and supplementary device orientations (e.g. certain axis of smartwatch and smartphone may be aligned during call only). The method subsequently proceeds to step 150 of determining a (second) characteristic of movement based on the obtained supplementary movement data.

After obtaining the first and second characteristics of movement, the method undertakes the step 160 of determining if the smartwatch is worn on the subject's dominant limb during the identified time period.

Here, step 160 comprises the step 170 of comparing the first and second characteristics of movement to determine a measure of similarity between the first and second characteristics of movement. Specifically, determining a measure of similarity comprises determining at least one of: correlation coefficients; rank correlation coefficients; a sum of absolute differences; a norm of an acceleration vector; timestamps of movement events; and an orientation of a three-dimensional vector. Based on the result of the comparison, the method proceeds to either step 180 or step 190.

Responsive to determining (in step 170) that the measure of similarity is above a predetermined threshold value (i.e. is a high value), the method proceeds to step 180 wherein it is determined that smartwatch is worn on the subject's dominant wrist/hand during the identified time period. Conversely, if it is determined (in step 170) that the measure of similarity is below a predetermined threshold value (i.e. is a low value), the method proceeds to step 190 wherein it is determined that smartwatch is worn on the subject's non-dominant wrist/hand during the identified time period (i.e. not worn on the subject's dominant wrist/hand).

From both step 180 and step 190, the method proceeds to step 200, wherein an operation or behavior of the smartwatch is modified based on the result of determining if the wearable device is worn on the subject's dominant limb during the identified time period. Here, the display of information by the smartwatch is configured to be dependent on whether or not it is worn on the dominant wrist/hand of the subject. Also, the health monitoring application provided by the smartwatch is configured according to the result of determining if the wearable device is worn on the subject's dominant limb. For instance, a parameter of a detection or monitoring function is adapted to account for the smartwatch not being worn on a subject's dominant wrist.

It will be appreciated that the above described exemplary embodiment of Figure 4 exploits the fact that a smartphone is carried by the subject and employs the proposal that dominant arm is statistically likely to be the arm that is used to perform an action with the smartphone. It is also noted that this can be quantified by looking at time similarity between smartphone movement and wrist/hand movement in a time window just before (i.e. immediately preceding) the smartphone is unlocked by the subject (e.g. a time window five seconds immediately before the unlocking action is performed). Because an accelerometer is provided within the smartphone, both the time of the unlocking action and accelerometer signals are available.

Here, the motion of the wrist where the smartwatch is worn is compared to the movement of the smartphone without assuming it to be representative of the whole body movement of the subject, thus focusing on the identification/detection of a specific gesture. Accordingly, in the embodiment of Figure 4, high similarity/correlation between movement of the smartwatch and the smartphone is proposed to be indicative of the subject wearing the smartwatch on the dominant arm, based on the proposal that, because the supplementary device is a smartphone, it will be more often used to perform actions on with the smartphone using the subject's dominant arm. Further, it is proposed that, for two-handed use of a smartphone, the hand holding the smartphone is more likely to be the non-dominant hand, while the dominant hand is performing actions on the smartphone (e.g. typing, pressing, tapping, swiping, etc.). Embodiments, may therefore be configured to distinguish between one-handed and two-handed use of the smartphone, e.g. based on detected movement and/or operations of the smartphone, and then consider the characteristic(s) of detected movement differently for the different time-periods.

The determination of the similarity can be performed on any suitable device with a processing unit, which can therefore be the same as that hosting either the wearable device or the supplementary device motion sensors. Depending on the chosen design for relative positioning of processing unit, the wearable device, and the supplementary device, signals can be transmitted from the wearable device and/or the supplementary device to the processing unit. The transmission of data might be synchronous, or asynchronous and triggered on a pre-set schedule, or in response to specific user behaviour for example.

In some embodiments, prior to the quantification of the measure of similarity between the wearable device and the supplementary device, periods when both the wearable device and the supplementary device are/were worn or carried can be separately identified.

An exemplary option for quantifying similarity between sensed movement of the wearable device and the supplementary device may include correlation coefficients, rank correlation coefficients, sum of absolute differences applied on the single axis acceleration signals, or three-dimensional accelerometer signals, or a signal derived from those, e.g. norm of a three-dimensional acceleration vector, orientation of a three-dimensional vector.

In an alternative approach, the similarity sensed movement of the wearable device and the supplementary device can be calculated as sum of absolute differences between timestamps of corresponding pairs landmark points (e.g. when the user takes out a smartphone from a pocket, a start and end of such movements can be identified based on signal characteristics such as signal power being above a fixed threshold or sudden change in orientation, and same can be done at the smartwatch). If the differences between timestamps are close in value (e.g. similar duration start-end of the movement measured at both devices), the acceleration signals may then be interpreted as being similar.

In another alternative embodiment, detection movement data can be compressed, summarized, or aggregated on the wearable device prior to be being provided (e.g. communicated) for analysis. Such summarization may include, calculating statistics over periods of time e.g. summing over one second windows, calculating variance over fifteen minute intervals. Put another way, various amounts of pre-processing of the movement and/or supplementary data may be undertaken by the wearable device and/or the supplementary device before being provided for analysis according to an embodiment. In this way, processing requirements may be located at various positions within a system according to an embodiment, depending on available capabilities and/or resources throughout the system for example.

Furthermore, embodiments may be repeatedly implemented (e.g. every hour, day or week) to repeatedly determine if a wearable device is worn on a subject's dominant limb. In this way, multiple determinations may be obtained and combined into a final (i.e. collective) determination for an extended time period of interest (e.g. a day combining hourly information).

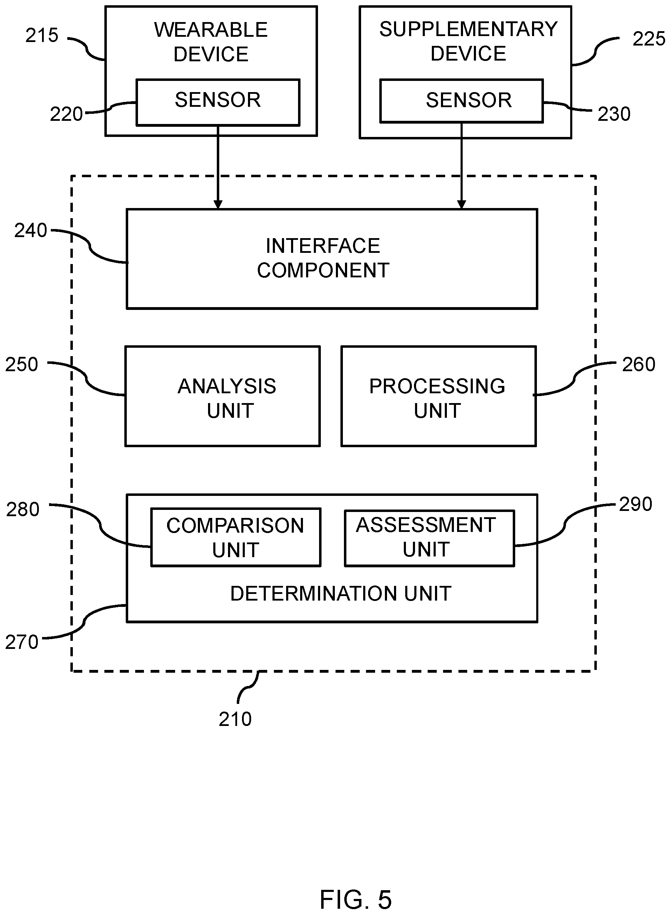

By way of further illustration of the proposed concept(s), a system for determining if a wearable device is worn on a subject's dominant limb according to another embodiment will be now be described with reference to Figure 5.

Figure 5 is a simplified block diagram of a system 210 for determining if a wearable device 215 is worn on a subject's dominant limb according to an embodiment.

In this embodiment, the wearable device 215 is a smart sports sock implementing an activity measurement algorithm and comprises a motion sensor 220. More specifically, the motion sensor 220 comprises a (first) accelerometer arrangement configured to generate movement data representative of sensed movement of the smart sports sock 215. Thus, the method of this embodiment is for determining if the smart sports sock is worn on the subject's dominant foot. This determination can subsequently be used to modify associated data for improved reliability and/or accuracy of activity data.

Further, the subject wears a supplementary device 225 around his/her torso, which in this example is a 'tracking vest' comprising a (supplementary) motion sensor 230 and communication interface (not shown) for communicating signals from the (supplementary) motion sensor 230 to the system 210. More specifically, the motion sensor 230 comprises a (second) accelerometer arrangement configured to generate supplementary movement data representative of sensed movement of the tracking vest 225.

The system 210 comprises an interface component 240 configured to obtain both movement data representative of movement of the smart sports sock device 215 and supplementary movement data representative of movement of the tracking vest 225.

The system 210 also comprises an analysis unit 250 that is configured to analyze (i.e. process with an algorithm) the movement data and the supplementary movement data in order to identify a time period of interest during which the subject is wearing both the smart sports sock 215 and the tracking vest 225.

A processing unit 260 of the system 210 is then configured to determine a first characteristic of movement based on the obtained movement data, and to determine a second characteristic of movement based on the obtained supplementary movement data. Put another way, the processing unit 260 determines: (i) a (first) characteristic of movement of the smart sport sock 215: and (ii) a (second) characteristic of movement of the tracking vest 225.

The system 210 further comprises a determination unit 270 that is configured to determine, based on a comparison of the first and second characteristics of movement, if the smart sports sock 215 is worn on the subject's dominant foot during the identified time period.

In this example, the determination unit 270 comprises a comparison unit 280 and an assessment unit 290.

The comparison unit 280 is configured to compare the first and second characteristics of movement to determine a measure of similarity between the first and second characteristics of movement.

The assessment unit 290 is then configured to determine if the smart sports sock 215 is worn on the subject's dominant foot during the identified time period based on the determined measure of similarity. More specifically, the assessment unit 290 of this example determines the smart sports sock 215 is worn on the subject's dominant limb during the identified time period if the determined measure of similarity is below a predetermined threshold value. This embodiment is thus based on the proposal that the subject will use one of his/her feet for instrumental, goal-oriented movement (e.g. kicking a ball, used as a pivot for turning, etc.), whereas, when the subject's entire body or torso moves (e.g. during a walk, travelling), both the dominant and non-dominant legs, and as a result feet, will also move (thus undergoing similar or highly correlated movement to that of the subject's body/torso). Based on such a proposal, it is inferred from a low measure of similarity that the smart sports sock 215 is worn on the subject's dominant limb during the identified time period.

From the above description of exemplary embodiment, it will be understood that there is proposed a concept which determines if a wearable device is worn on the dominant or non-dominant limb of a subject. Such a concepts may be based on comparing sensed movements of the wearable device being worn by the user with sensed concurrent (i.e. simultaneous) movements of another, supplementary device worn or carried by the subject.

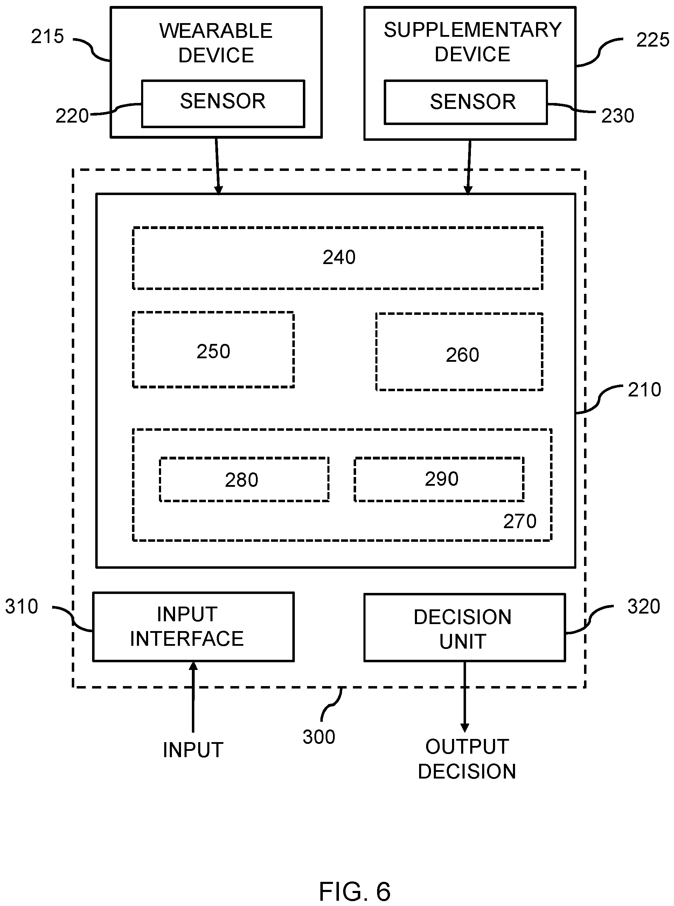

Also, it is proposed that such a determination about whether a subject is wearing a wearable device on the dominant or non-dominant limb (as provided by proposed embodiments) may be combined with a user-provided indication (or an automatically detected current wearing position (left/right foot) to infer the general footedness (i.e. general preference for left or right) of the subject. To further illustrate such a proposal, a system for determining a subject's limb preference for wearing a wearable device will now be described with reference to Figure 6.

Figure 6 illustrates a system 300 for determining a subject's limb preference for wearing a wearable device according to a proposed embodiment. Here, the system is used in conjunction with the wearable device 215 and supplementary device 225 of the embodiment of Figure 5.

The system 300 comprises a system 210 for determining if a wearable device is worn on a subject's dominant limb according to the embodiment of Figure 5. The system also comprises an input interface 310 and a decision unit 320.

The input interface is configured to obtain an indication of a current wearing position of the wearable device on the subject. In this example, such an indication is provided to the input interface by the subject as a user input signal (labelled "INPUT" in Figure 6).

As been described above with reference to Figure 5, the system 210 determines if the wearable device 215 is worn on the subject's dominant limb. The decision unit 320 is then configured to determine the subject's limb preference for wearing the wearable device based on: the result determined by the system 210 and the indication of a current wearing position of the wearable device obtained by the input interface 310.

In this way, results obtained by the embodiment of Figure 5 may be used in combination with extra information to provide a more reliable and/or accurate determination of a subject's limb preference. Thus, there may be provided an automatic way to obtain an accurate indication of a subject's limb preference without relying on potentially inaccurate or misleading information provided by the subject (e.g. in response to a questionnaire).

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

## Claims

1. A computer-based method for determining if a wearable device is worn on a subject's dominant limb, the wearable device comprising a motion sensor configured to generate movement data representative of movement of the wearable device, the method comprising:
identifying (110) a time period of interest during which the subject is both: wearing the wearable device; and carrying a supplementary device comprising a supplementary motion sensor configured to generate supplementary movement data representative of movement of the supplementary device;
obtaining (120) movement data representative of movement of the wearable device during the identified time period;
obtaining (130) supplementary movement data representative of movement of the supplementary device during the identified time period;
determining (140) a first characteristic of movement based on the obtained movement data;
determining (150) a second characteristic of movement based on the obtained supplementary movement data; and
based on a comparison of the first and second characteristics of movement, determining (160) if the wearable device is worn on the subject's dominant limb during the identified time period.

2. The method of claim 1, wherein determining if the wearable device is worn on the subject's dominant limb during the identified time period comprises:
comparing (170) the first and second characteristics of movement to determine a measure of similarity between the first and second characteristics of movement; and
determining if the wearable device is worn on the subject's dominant limb during the identified time period based on the determined measure of similarity.

3. The method of claim 2, wherein determining if the wearable device is worn on the subject's dominant limb during the identified time period comprises:
determining (180) the wearable device is worn on the subject's dominant limb during the identified time period if the determined measure of similarity is below a predetermined threshold value.

4. The method of claim 2 or 3, wherein determining a measure of similarity comprises determining at least one of:
correlation coefficients;
rank correlation coefficients;
a sum of absolute differences;
norm of vectors;
differences in timestamps of movement events;
orientation of a three-dimensional vectors; and
differences in orientation of three-dimensional vectors.

5. The method of any of claims 1 to 4, wherein identifying (110) a time period of interest comprises: identifying (114) the time period of interest responsive to the occurrence of a predetermined action performed by the subject.

6. The method of claim 5, wherein the supplementary device comprises a portable computing device, and wherein the predetermined action comprises a gesture using an input interface of the computing device.

7. The method of any of claims 1 to 6, wherein the characteristic of movement comprises at least one of: amplitude of movement; direction of movement; frequency of movement; speed of movement; variation in position; variation in angle; and variance rate.

8. The method of any of claims 1 to 7, further comprising:
based on the result of determining if the wearable device is worn on the subject's dominant limb during the identified time period, modifying (200) an operation or behavior of the wearable device.

9. The method of any of claims 1 to 8, wherein the motion sensor comprises a first accelerometer arrangement, and wherein the supplementary motion sensor comprises a second accelerometer arrangement.

10. A computer-based method for determining a subject's limb preference for wearing a wearable device, the method comprising:
determining if a wearable device is worn on the subject's dominant limb according to any of claims 1 to 8;
obtaining an indication of a current wearing position of the wearable device on the subject; and
determining the subject's limb preference for wearing a wearable device based on: the result of determining if the wearable device is worn on the subject's dominant limb; and the obtained indication of a current wearing position of the wearable device on the subj ect.

11. A computer program comprising code means for implementing the method of any of claims 1 to 10 when said program is run on a processing system.

12. A system (210) for determining if a wearable device (215) is worn on a subject's dominant limb, the wearable device comprising a motion sensor (220) configured to generate movement data representative movement of the wearable device, the system comprising:
an analysis unit (250) configured to identify a time period of interest during which the subject is both: wearing the wearable device (215); and carrying a supplementary device (225) comprising a supplementary motion sensor (230) configured to generate supplementary movement data representative of movement of the supplementary device;
an interface component (240) configured to obtain movement data representative of movement of the wearable device during the identified time period, and to obtain supplementary movement data representative of movement of the supplementary device during the identified time period;
a processing unit (260) configured to determine a first characteristic of movement based on the obtained movement data, and to determine a second characteristic of movement based on the obtained supplementary movement data; and
a determination unit (270) configured to determine, based on a comparison of the first and second characteristics of movement, if the wearable device is worn on the subject's dominant limb during the identified time period.

13. The system of claim 12, wherein determination unit (270) comprises:
a comparison unit (280) configured to compare the first and second characteristics of movement to determine a measure of similarity between the first and second characteristics of movement; and
an assessment unit (290) configured to determine if the wearable device is worn on the subject's dominant limb during the identified time period based on the determined measure of similarity.

14. The system of claim 13, wherein the assessment unit (290) is configured to determine the wearable device is worn on the subject's dominant limb during the identified time period if the determined measure of similarity is below a predetermined threshold value.

15. A system (300) for determining a subject's limb preference for wearing a wearable device, the system comprising:
a system (210) for determining if a wearable device is worn on the subject's dominant limb according to any of claims 12 to 14;
an input interface (310) configured to obtain an indication of a current wearing position of the wearable device on the subject; and
a decision unit (320) configured to determine the subject's limb preference for wearing a wearable device based on: the result of determining if the wearable device is worn on the subject's dominant limb; and the obtained indication of a current wearing position of the wearable device on the subject.
